# EUROPEAN PATENT APPLICATION

(11) **EP 2 348 013 A1**
(43) Date of publication of application: **27.07.2011**
(21) Application number: 10151583.1
(22) Date of filing: 25.01.2010
(51) Int. Cl.: C07C 213/04, C07C 217/70, C07C 233/43

(54) **Process for preparation of intermediates of arformoterol**

(71) Applicant: INKE, S.A., 08755 Castellbisbal (ES)
(72) Inventor: Navarro Muñoz, Isabel, 08970 Sant Joan Despi (ES); Huguet Clotet, Juan, 08970 Sant Joan Despi (ES)
(74) Representative: Ponti Sales, Adelaida

(57) **Abstract**

An improved method for the preparation of optically pure isomers of Formoterol is disclosed, particularly the (R,R)-isomer.

A method of preparation of substantially enantiomerically pure (R,R)-1-(4-Benzyloxy-3-nitro-phenyl)-2-[[2-(4-methoxy-phenyl)-1-methyl-ethyl]-(1-phenyl-ethyl)-amino]-ethanol to use in the production of (R,R)-Formoterol is also disclosed

## Description

### Field of invention

The present invention relates to an enantioselective process for the preparation of intermediates of Arformoterol, and their use in the preparation of Arformoterol or an acid addition salt thereof, which is useful as a bronchodilatator.

### Background of the invention

Formoterol, (+/-)N-[2-hydroxy-5-[1-hydroxy-2-[[2-(p-methoxyphenyl)-2-propylamino]ethyl]phenyl]-formamide, is a highly potent and β₂-selective adrenoceptor agonist having a long lasting bronchodilating effect when inhaled. Its chemical structure is depicted below:

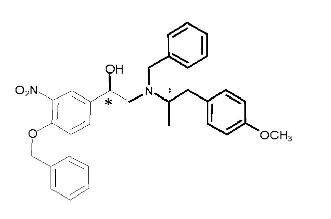

Formoterol has two chiral centres, each of which can exist into two different configurations. This results into four different combinations, (R,R), (S,S), (S,R) and (R,S). Formoterol is commercially available as a racemic mixture of 2 diasteromers (R,R) and (S,S) in a 1:1 ratio. The generic name Formoterol always refers to its racemic mixture. *Trofast et al.* (Chirality, 1, 443, 1991) reported on the potency of these isomers, showing a decrease in the order of (R,R)>(R,S)≥(S,R)>(S,S). The (R,R) isomer, also known as Arformoterol, being 1000 fold more potent than the (S,S) isomer. Arformoterol is commercialised by Sepracor as Brovana.

Formoterol was first disclosed in Japanese patent application (Application N° 13121) whereby Formoterol is synthesised by N-alkylation using a phenacyl bromide as described in the scheme below:

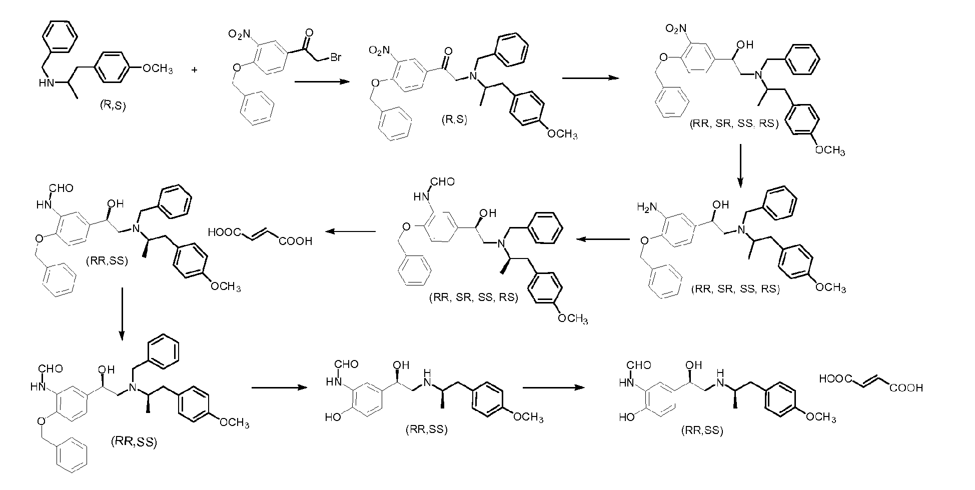

Afterwards, a small number of methods have been reported so far, regarding the synthesis of the (R,R) isomer, also referred as (R,R)-Formoterol and Arformoterol.

Murase et al. [Chem. Pharm. Bull. 26(4) 1123-1129(1978)] reported the preparation of (R,R)-Formoterol from a racemic mixture of the (R,R) and (S,S) isomers by optical resolution using optically active tartaric acid. Trofast *et al.* described a method in which 4-benzyloxy-3-nitrostyrene oxide was coupled with a optically pure (R,R)- or (S,S)-N-phenylethyl-N-(1-p-methoxyphenyl)-2-(propyl)amine to give a diastereomeric mixture of Formoterol precursors. These precursors were further separated by HPLC in order to obtain pure Formoterol isomers. Both synthetic processes undergo long synthetic procedures and low yields.

Patent publication EP0938467 describes a method in which Arformoterol is prepared *via* the reaction of the optically pure (R) N-benzyl-2-(4-methoxyphenyl)-1-(methylethylamine) with an optically pure (R)-4-benzyloxy-3-nitrostyrene oxide or (R)-4-benzyloxy-3-formamidostyrene oxide followed by formylation of the amino group. This method requires relatively severe reaction conditions, 24 h at a temperature of from 110 up to 130 °C as well as a further purification step using tartaric acid in order to eliminate diastereomer impurities formed during the process.

WO2009/147383 discloses a process for the preparation of intermediates of Formoterol and Arformoterol which comprises a reduction of a ketone intermediate of formula:

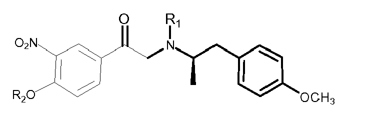

Using chiral reductive agent with an enantiomeric excess of about 98% which requires further purification steps to obtain a product of desired optical purity.

Therefore, it is still necessary to develop an industrialisable process which provides intermediates with high optical purity in an efficient manner.

### Summary of the invention

The present invention relates to a process for the preparation of a compound of formula IV 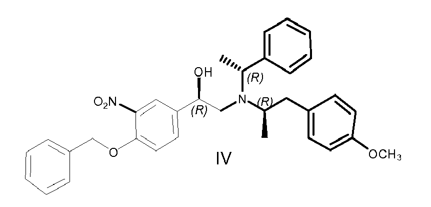 which comprises converting compound of formula I 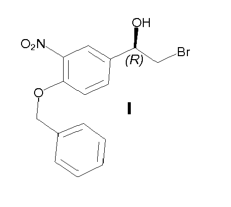 into a compound of formula II 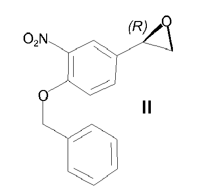 and reacting the compound of formula II with a compound of formula III

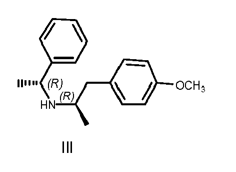

A second aspect of the invention relates to a process for preparing optically pure (R,R)-Formoterol (Arformoterol, also referred as to compound VII) or a salt thereof from optically pure and industrially stable intermediate of formula IV in good yields, comprising the further steps of:
a) reducing the nitro group of a compound of formula IV to obtain a compound of formula V 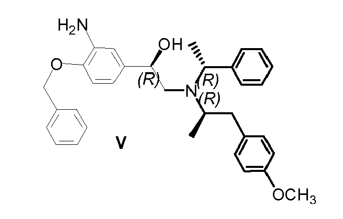
b) formylating of the primary amino group of the compound V to obtain a compound of formula VI. 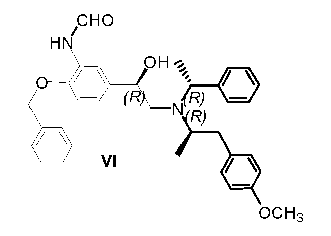
c) debenzylating the compound of formula VI by hydrogenolysis, in the presence of a metal catalyst, to generate the compound VII. 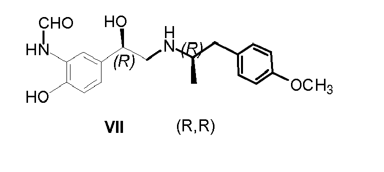
d) optionally, subjecting the obtained compound of formula VII to salt formation.

Furthermore, the invention relates to the preparation of compounds of formula V and VI.

In an other aspect the invention relates to a pharmaceutical composition comprising (R,R)-Formoterol obtained by the process of the invention, together with one or more pharmaceutical acceptable excipients.

### Detailed description of the invention

The process of the invention provides an enantioselective process for preparing optically pure (R,R)-Formoterol (Arformoterol) or a salt thereof from optically pure and stable intermediate (R)-2-(4-Benzyloxy-3-nitro-phenyl)-oxirane (compound II), suitable for industrial use, in combination with optically pure amine in higher yields, as depicted in the scheme below:

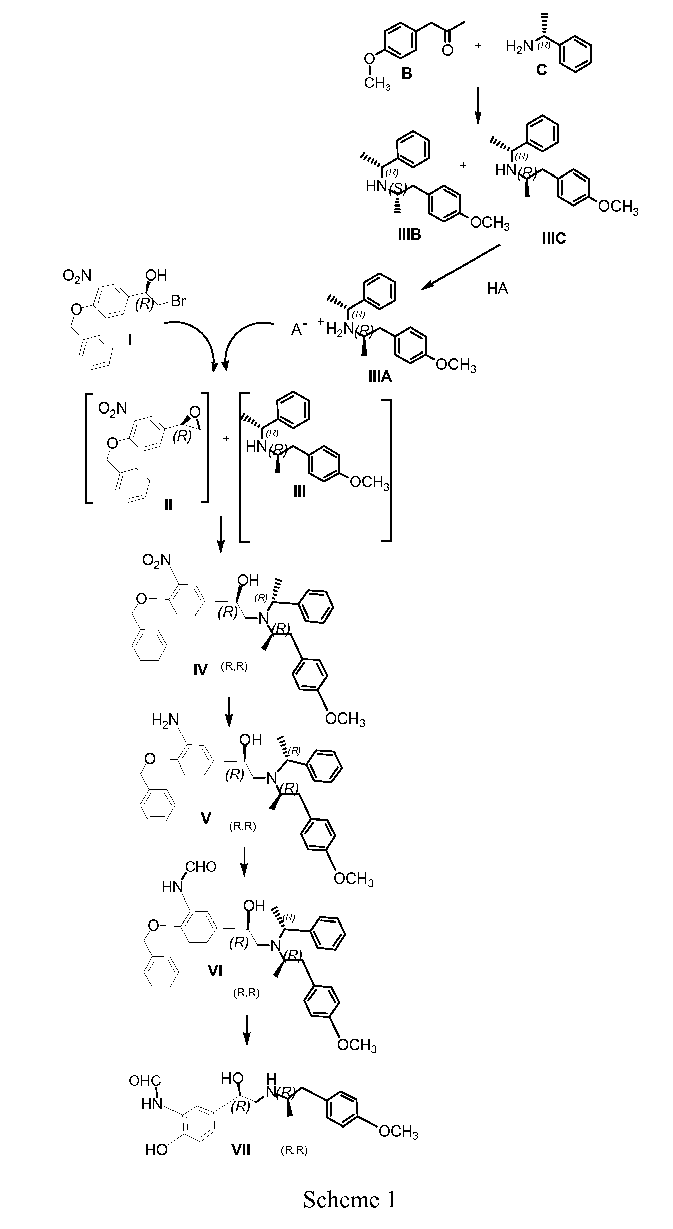

The (R,R) configuration of chiral centres indicated in Scheme 1 refers to the chiral centres present in the molecule of Arformoterol. Compounds in brackets also indicate that said compounds could be isolated within the integrated process.

In the present invention, enantiomerically pure or substantially enantiomerically pure is meant to be at least 99.95% of optical purity measured by chiral HPLC.

A first aspect of the invention relates to a process for the preparation of a compound of formula IV which comprises, converting a compound of formula I 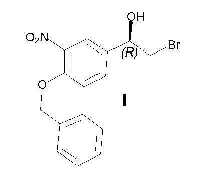 into an oxirane of formula II in presence of an organic solvent or mixtures thereof.

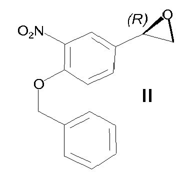

The compound of formula I can be obtained following known methods as described by Wilkinson, H.S et al., (Organic Process Research and Development 2002,6, 146-148).

The oxirane compound of formula II is subsequently reacted with the amine III.

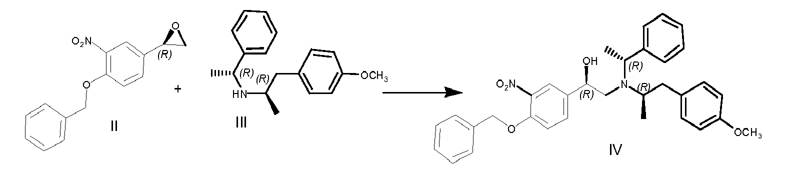

The reaction is performed directly, in absence of a solvent under nitrogen atmosphere at a temperature range from 20 ° to 150 °C. The reaction yields the desired (R,R) isomer with an optical purity of 100%, hence with no formation of the other possible isomers.

The amine of formula III can be also generated *in-situ* from an acid salt of [2-(4-Methoxyphenyl)-1-methyl-ethyl]-(1-phenyl-ethyl)-amine to further react with the oxirane compound of formula II yielding compound IV. The acid salt is an organic or an inorganic base. Preferably, organic salts are non-chiral salts such as maleates and citrates. Preferably, inorganic salts are prepared from acids of the type of HA, where A represents a halogen. Most preferably the inorganic salt is hydrochloride.

The amine of formula III can be prepared *in-situ* from an acid salt of [2-(4-Methoxyphenyl)-1-methyl-ethyl]-(1-phenyl-ethyl)-amine, in the presence of an organic or inorganic base, a protic solvent and a co-solvent. Protic solvent are alcohols, water and mixtures thereof. Co-solvent can be selected form organic solvents such as, linear, branched and cyclic hydrocarbons, ketones and alkyl acetates. Preferably, the co-solvent is toluene. The solvent media will be eliminated once the formation of all the free amine is completed, thus the already formed amine will react with the epoxide of formula II. Inorganic bases can be selected from the group of: carbonates, bicarbonates and hydroxides of alkali or alkaline earth metals and ammonia. Organic bases are of the type of lower alkyl amines.

Optionally, compound of formula I can be transformed directly into compound IV from the reaction of (R)-1-(4-Benzyloxy-3-nitro-phenyl)-2-bromo-ethanol (compound I) and (R,R)-[2-(4-Methoxy-phenyl)-1-methyl-ethyl]-(1-phenyl-ethyl)-amine (compound III), whereby the epoxide (compound II) is formed *in-situ* and does not require the isolation step of compound II.

The obtained compound IV can be isolated.

It has been surprisingly found that the use of phenyethyl moiety as protecting group increases the yield in the preparation of Arformoterol compared with other processes performed using other protecting groups. As a result of that, compound IV is obtained in high purity, allowing the use of said compound, directly, in following reactions without the need of further purification.

Furthermore, the use of the phenylethyl protecting group, allows an improved preparation of amine III without the need to use chiral acids. By using (R)-phenylethylamine, the resulting amine III has two chiral centres, also referred throughout the present application as a bi-chiral amine. This facilitates the preparation compound III, allowing the use of any non-chiral acid to form a salt which can be easily precipitated to obtain the desired diastereoisomer. Acids that can be used are usually inorganic or organic acids, such as hydrochloric acid, hydrobromic acid, sulphuric acid, phosphoric acid, acetic acid, preferably hydrochloric acid. Furthermore, the use of a bi-chiral amine also increases significantly the overall yield as compared to using a chiral salt such as mandelate in order to obtain the desired enantiomers. Prior art shows lower yields in comparison with the process described herein.

A second aspect of the invention relates to a process for the preparation of Arformoterol using a compound of formula IV obtained by the processes described above. The synthesis of Arformoterol comprises the steps of:
a) reducing the nitro group of the compound of formula IV to obtain a compound of formula V

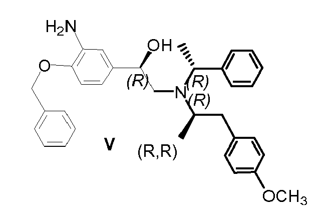

The reduction is carried out in the presence of a metal catalyst and an organic solvent or mixture of solvents. The metal catalyst can be selected from nickel-Raney, palladium or platinum based catalyst. Suitable organic solvents include alcohols, ethers and linear, branched or cyclic hydrocarbons and mixtures thereof. Preferably, alcohols such as isopropanol (IPA) and hydrocarbons such as toluene. The Compound V can be purified or used in further steps directly, without purification.
b) formylating of the primary amino group of compound V using acetic formic anhydride in the presence of an organic solvent to obtain a compound of formula VI.

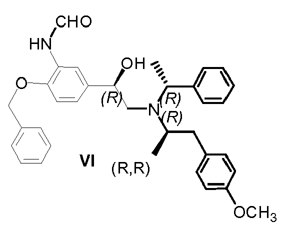

Acetic formic anhydride, could be prepared by mixing acetic anhydride and formic acid. Suitable organic solvents include chlorinated solvents, linear, cyclic and branched hydrocarbons and mixtures thereof. Compound VI can be purified or used in further steps directly, without purification.
c) debenzylating compound of formula VI by hydrogenolysis, in the presence of a metal catalyst and an organic solvent to generate compound VII. The hydrogenation is carried out in the presence a noble metal catalyst selected from palladium or platinum based catalyst. Organic solvents are preferably alcohols, ethers, esters, ketones and linear, branched or cyclic hydrocarbons and mixtures thereof.
d) optionally, subjecting the obtained compound of formula VII to salt formation. Preferably, Arformoterol tartrate.

Arformoterol tartrate prepared by the process of the invention can be crystallised as Arformoterol form A, as described in Pat. No. EP1082293. The Arformoterol form A obtained can be further subjected to milling or grinding.

Furthermore, the present invention also relates to the preparation of compounds of formula V and VI. Methods to prepare said compounds are described below:

The compound (R, R)-1-(4-Benzyloxy-3-nitro-phenyl)-2-[[2-(4-methoxy-phenyl)-1-methyl-ethyl]-(1-phenyl-ethyl)-amino]-ethanol (compound V), having a configuration represented by the following formula: 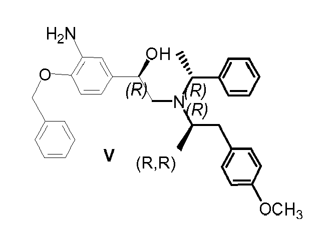 is prepared by the reduction of the nitro group of the compound IV in the presence of a metal catalyst and a solvent or mixture of solvents. The reduction is carried out in the presence of a metal catalyst selected from nickel-Raney, palladium catalyst and platinum based catalyst. The reaction is performed in heterogeneous phase.

The term "palladium catalyst" is taken to mean a palladium compound, such as Pd/C. The term "platinum catalyst" is taken to mean a platinum compound, such as Pt/C.

Suitable organic solvents include alcohols, ethers and linear, branched or cyclic hydrocarbons and mixtures thereof. Preferably, alcohols such as isopropanol and hydrocarbons such as toluene. The reaction is carried out between 25 and 60 °C and at high pressure.

The compound V can be purified or used in further steps directly, without isolation or purification.

Compound (R, R)-1-(4-Benzyloxy-3-nitro-phenyl)-2-[[2-(4-methoxy-phenyl)-1-methylethyl]-(1-phenyl-ethyl)-amino]-ethanol (compound VI), having the configuration represented by the following formula: 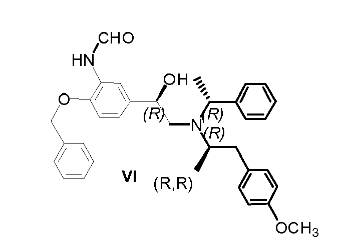 is prepared by the formylation of the primary amino group of the compound V using acetic formic anhydride and formic acid and an organic solvent or mixtures of solvents. Suitable organic solvents include chlorinated solvents, linear, cyclic and branched hydrocarbons and mixtures thereof. The reaction takes place from -10 °C up to 25 °C.

The Compound VI can be purified or used in further steps directly, without isolation or purification.

In another aspect, the invention relates to a pharmaceutical composition comprising R,R-Formoterol according the process of the invention, together with one or more pharmaceutically acceptable excipients.

### Examples

### (R)-2-(4-Benzyloxy-3-nitro-phenyl)-oxirane (II)

A solution of 90 g (0.25 mol) of (R)-1-(4-Benzyloxy-3-nitro-phenyl)-2-bromo-ethanol (compound I) in 320 mL of toluene and 50 mL of MeOH was added to a stirred suspension of 46 g (0.33 mol) of K₂CO₃ in 130 mL of toluene and 130 mL of MeOH. The mixture was stirred at 40°C for 20 h and washed with water (400 mL). The organic phase was concentrated under reduced pressure to a volume of 100 mL and stirred at 25 °C for 30 min. It was then further cooled to 0-5°C for 30 min. and the product collected by filtration and dried at 40 °C to provide 67.1 g (97% yield) (98% chemical purity, 100% e.e.) of compound II as an off-white solid. ¹**H-NMR (200 MHz, CDCl₃) δ:** 2.80-2.90 (m, 2H); 3.11-3.20 (m, 2H), 3.80-3.90 (m, 1H); 5.23 (s, 2H); 7.11 (d, 2H); 7.41 (m, 5H), 7.76 (d, 2H).

### Preparation of (R,R)-[2-(4-Methoxy-phenyl)-1-methyl-ethyl]-(1-phenyl-ethyl)-amine (III)

A solution of 13 g (78.6 mmol) of 1-(4-Methoxy-phenyl)-propan-2-one and 8.3 g (78.6 mmol) of (R)-1-Phenylethylamine in 60 mL MeOH was hydrogenated in the presence of 1.7 g of Pt/C 5% at 10 atm. and 30 °C for 20 h. The mixture was filtered though a pad of diatomaceous earth and concentrated under reduced pressure to give compound III as an oil. The obtained oil was dissolved in 175 mL of acetone, followed by addition of 6.7 mL (80.9 mmol) of a 12M HCl solution. The mixture was stirred at 23 °C for 30 min and at 0-5 °C for 30 min. The product collected by filtration and dried at 40 °C to provide 13.8 g of the hydrochloride derivate as a white solid. The obtained solid was stirred in 100 mL of acetone at 23 °C for 1h and at 0-5 °C for 30 min, collected by filtration and dried at 40 °C to provide 13.2 g of the hydrochloride derivate as a white solid. This compound was dissolved in 100 mL of water and 100 mL of toluene followed by addition of 54 mL (54 mmol) of 1N NaOH solution. The organic phase was concentrated to give 11.7 g (55% yield) (99% chemical purity and 100% e.e) of compound III as an oil. **¹H-NMR (200 MHz, CDCl₃) δ:** 0.88 (d, 3H); 1.31 (d, 3H), 2.40-2.50 (m, 1H); 2.60-2.80 (m, 2H); 3.74 (s, 3H); 3.90-4.10 (m, 1H); 6.77- 6.98 (m, 4H), 7.31 (s, 5H).

### Synthesis of (R,R)-1-(4-Benzyloxy-3-nitro-phenyl)-2-[[2-(4-methoxy-phenyl)-1-methyl-ethyl]-(1-phenyl-ethyl)-amino]-ethanol (IV)

A 1-liter flask was charged with 50g (0.18 mol) of **II** and 50g (0.18 mol) of **III** and stirred under nitrogen atmosphere at 140 °C for 20 h. To the hot mixture was added 200 mL of toluene to obtain a solution, which was washed with 200 mL of 1N HCl and 200 mL of water. The organic phase was concentrated under reduced pressure to give 99 g (99% yield) (88% chemical purity) of compound IV as an oil. Enantiomeric purity 100%. **¹H-NMR (200 MHz, CDCl₃) δ:** 0.98 (d, 3H); 1.41 (d, 3H), 2.60-2.90 (m, 4H); 3.20-3.30 (m, 1H); 3.74 (s, 3H); 4.10-4.20 (m, 1H); 4.30-4.40 (m, 1H), 5.19 (s, 2H); 6.69-7.42 (m, 16H); 7.77 (s, 1H).

### Synthesis of (R, R)-1-(3-Amino-4-benzyloxy-phenyl)-2-[[2-(4-methoxy-phenyl)-1-methyl-ethyl]-(1-phenyl-ethyl)-amino]-ethanol (V)

A solution of 99 g (0.18 mol) of **IV** in 270 mL IPA and 270 mL toluene was hydrogenated in the presence of 10 g of Ni-Raney at 18 atm and 40 °C for 20 h. The mixture was filtered though a pad of diatomaceous earth and the filtrate was concentrated under reduced pressure to give 87 g (92% yield) (83% chemical purity, 100 % e.e.) of compound **V** as an oil. **¹H-NMR (200 MHz, CDCl₃) δ**: 0.97 (d, 3H); 1.44 (d, 3H), 2.60-2.90 (m, 4H); 3.20-3.30 (m, 1H); 3.74 (s, 3H); 4.10-4.20 (m, 1H); 4.30-4.40 (m, 1H), 5.07 (s, 2H); 6.67-6.84 (m, 7H); 7.25-7.42 (m, 10H).

### Synthesis of (R,R)-N-(2-Benzyloxy-5-{1-hydroxy-2-[[2-(4-methoxy-phenyl)-1-methyl-ethyl]-(1-phenyl-ethyl)-amino]-ethyl)-phenyl)-formamide (VI)

24 mL (0.63 mol) of formic acid was added to 27 mL (0.28 mol) of acetic anhydride and stirred at 50 °C for 2 h under nitrogen atmosphere. The resulting mixture was diluted with 100 mL of CH₂Cl₂ and cooled to 0 °C. A solution of 78 g (0.15 mol) of **V** in 300 mL de CH₂Cl₂ was slowly added and stirred for 1h at 0 °C. Then, 150 mL of 10% K₂CO₃ aqueous solution were added and stirred at 0 °C for 15 min. The organic phase was washed twice with 400 mL of 10% K₂CO₃ aqueous solution and concentrated under reduced pressure to give 80 g (97% yield, 100% e.e.) (75% chemical purity) of compound **VI** as an oil. **¹H-NMR (200 MHz, CDCl₃) δ**: 0.98 (d, 3H); 1.42 (d, 3H), 2.60-2.90 (m, 4H); 3.20-3.30 (m, 1H); 3.75 (s, 3H); 4.10-4.20 (m, 1H); 4.30-4.40 (m, 1H), 5.09 (s, 2H); 6.67-7.41 (m, 17H); 8.4 (d, 1H).

### Synthesis (R,R)-N-(2-Hydroxy-5-{1-hydroxy-2-[2-(4-methoxy-phenyl)-1-methyl-ethylamino]-ethyl}-phenyl)-formamide (VII)

A solution of 8.5 g (16 mmol) of **VI**, previous purified by column chromatography on silica gel (AcOEt/heptane, 2:3), in 60 mL ethanol was hydrogenated in the presence of 0.14 g of Pd/C 5% at 10 atm. and 40 °C for 20 h. The mixture was filtered though a pad of diatomaceous earth and concentrated under reduced pressure to give 5 g (93% yield) (91% chemical purity, 100% e.e.) of compound **VII** as foam. m. p.= 58-60 °C. **¹H-NMR (200 MHz, d₆-DMSO) δ**: 0.98 (d, 3H); 2.42-2.65 (m, 5H); 3.20-3.40 (m, 1H); 3.71 (s, 3H); 4.43-4.45 (m, 1H); 6.77-7.05 (m, 5H); 8.02 (s, 1H), 8.26 (s, 1H).

### Synthesis (R,R)-N-(2-Hydroxy-5-{1-hydroxy-2-[2-(4-methoxy-phenyl)-1-methyl-ethylamino]-ethyl}-phenyl)-formamide (VII)

A solution of 46 g (0.08 mol) of VI, crude product, was dissolved in 460 mL ethanol and hydrogenated in the presence of 0.74 g of Pd/C 5% at 10 atm. and 40 ° C for 28 h. The mixture was filtered though a pad of diatomaceous earth and the filtrate was concentrated under reduced pressure to give 24 g (83% yield) (77% chemical purity, 100% e.e.) of compound VII as a foam. m. p. = 58-60 °C. **¹H-NMR (200 MHz, d₆-DMSO) δ:** 0.98 (d, 3H); 2.42-2.65 (m, 5H); 3.20-3.40 (m, 1H); 3.71 (s, 3H); 4.43-4.45 (m, 1H); 6.77-7.05 (m, 5H); 8.02 (s, 1H), 8.26 (s, 1H).

The HPLC conditions used for the determination of the Chemical purity % are described in the table below:

| | |
|---|---|
| | |
| HPLC Column | Kromasil 100 C-18 |
| Dimensions | 0.15 m x 4.6 mm x 5 µm |
| Buffer | 2.8 ml TEA (triethylamine) pH=3.00 H₃PO₄ (85%) in 1 L of H₂O |
| Phase B | Acetonitrile |
| Flow rate | 1.5 ml miN⁻¹ |
| Temperature | 40 °C |
| Wavelength | 230 nm |

The HPLC conditions used for the determination of the enantiomeric purity % are described in the table below:

| | |
|---|---|
| | |
| HPLC Column | Chiralpak AD-H |
| Dimensions | 0.25 m x 4.6 mm |
| Buffer | n-hexane : IPA : DEA (diethyl amine) : H₂O 85:15:0.1:0.1 |
| Flow rate | 0.8 ml min⁻¹ |
| Temperature | 25 °C |
| Wavelength | 228 nm |

## Claims

1. Process for preparing a substantially enantiomerically pure compound of formula IV 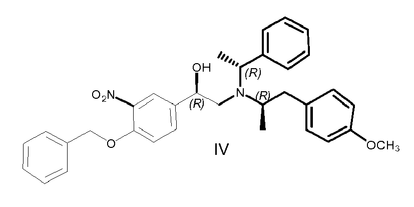 by reacting compound of formula II 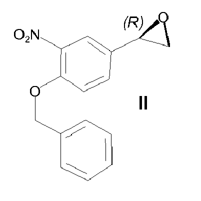 with compound of formula III 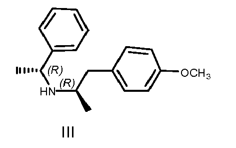 wherein the reaction takes place in absence of a solvent.

2. A process according to claim 1, wherein the compound of formula III is generated *in-situ* from a salt of compound III in the presence of an organic solvent and an organic or inorganic base, followed by the removal of the organic solvent.

3. A process according to claim 1, wherein the compound of formula IV is obtained directly by reacting compound III with compound I 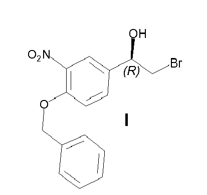 in the presence of an organic solvent or mixtures thereof, whereby compound of formula II is generated *in-situ.*

4. A process according to claim 1, wherein compound of formula II is isolated and purified.

5. A process according to claim 1, wherein the preparation of a compound of formula III, comprises reacting compound of formula B with a (R)-phenyethylamine (compound C) in the presence of a reducing agent and an organic solvent, followed by separation of the desired diastereomer using a non-chiral acid, followed by treatment with a base to obtain the compound of formula III in base form.

6. A process according to claim 5, wherein the non-chiral acid is an organic or inorganic acid, preferably hydrochloric acid.

7. A process according to claim 1, wherein compound of formula III is isolated and purified.

8. A process for the preparation of Arformoterol comprising the steps of:
a) reducing the nitro group of compound of formula IV prepared according to claims 1 to obtain compound of formula V 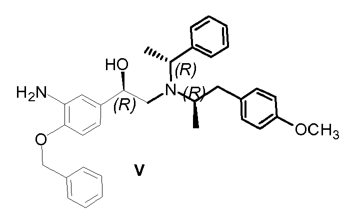
b) formylating of the primary amino group of compound V with acetic formic anhydride to obtain compound of formula VI. 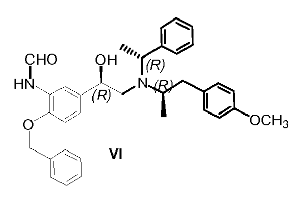
c) debenzylating compound of formula VI by hydrogenolysis, in the presence of a noble metal catalyst to generate compound VII
d) optionally, subjecting the obtained compound of formula VII to salt formation.

9. A process according to claim 8, wherein the reduction of the nitro group of compound IV is carried out using a metal catalyst selected from the group of Nickel-Raney, palladium and platinum based catalyst.

10. A process according to claim 9, wherein the reduction of the nitro group of compound IV is carried out in the presence of an organic solvent, selected from the group, consisting of alcohols, ethers, linear, branched or cyclic hydrocarbons and mixtures thereof. Preferably, alcohols such as isopropanol and hydrocarbons such as toluene.

11. A process according to claim 8, wherein the formulation of compound V is carried out in the presence of an organic solvent, selected from the group consisting of, chlorinated solvents, linear, cyclic and branched hydrocarbons and mixtures thereof.

12. A process according to claim 8, wherein the debenzylation of compound VI is carried out using a metal catalyst selected from the group of palladium based catalyst.

13. A process according to claim 12, wherein the debenzylation of compound VI is carried out in the presence of an organic solvent, selected from the group consisting of, alcohols, ketones, esters, ethers and mixtures thereof.

14. A process for producing enantiomerically pure (R, R)-1-(4-Benzyloxy-3-nitrophenyl)-2-[[2-(4-methoxy-phenyl)-1-methyl-ethyl]-(1-phenyl-ethyl)-amino]-ethanol (compound V), having a configuration represented by the following formula: 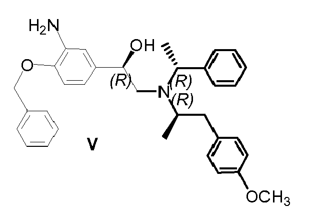 which comprises the reduction of the nitro group of compound IV obtained according to claims 1 to 6, in the presence of a metal catalyst and an organic solvent or mixture thereof.

15. A process for producing enantiomerically pure (R, R)-1-(4-Benzyloxy-3-nitrophenyl)-2-[[2-(4-methoxy-phenyl)-1-methyl-ethyl]-(1-phenyl-ethyl)-amino]-ethanol (compound VI), having a configuration represented by the following formula: 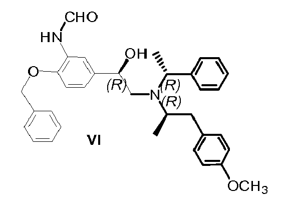 which comprises the formylation of the primary amino group of compound V obtained according claim 8, with acetic formic anhydride in the presence of an acid and an organic solvent or mixture thereof.
